(19) **Europäisches Patentamt European Patent Office Office européen des brevets**

(11) **EP 1 882 520 B1**

(12) **EUROPEAN PATENT SPECIFICATION**

(45) Date of publication and mention of the grant of the patent:
**26.05.2010 Bulletin 2010/21**

(51) Int Cl.:
***B01J 19/00*** *(2006.01)*

(21) Application number: **07101575.4**

(22) Date of filing: **01.02.2007**

(54) **Method of manufacturing a patterned spot microarray using a photocatalyst and the microarray produced thereby**

Herstellungsverfahren für Mikroarray mit gemusterten Spots und Photokatalysator sowie dadurch hergestelltes Mikroarray

Procédé de fabrication des micro-réseaux structurés en appliquant un photocatalyseur ainsi que les micro-réseaux obtenus selon ce procédé

(84) Designated Contracting States:
**DE GB**

(30) Priority: **25.07.2006 KR 20060069797**

(43) Date of publication of application:
**30.01.2008 Bulletin 2008/05**

(73) Proprietor: **Samsung Electronics Co., Ltd. Suwon-si, Gyeonggi-Do (KR)**

(72) Inventors:
• **Chung, Jong-Suk Giheung-gu, Yongin-si, Gyeonggi-do (KR)**
• **Hwang, Kyu-youn Giheung-gu, Yongin-si, Gyeonggi-do (KR)**
• **Shim, Jeo-young Giheung-gu, Yongin-si, Gyeonggi-do (KR)**

(74) Representative: **Grünecker, Kinkeldey, Stockmair & Schwanhäusser Anwaltssozietät Leopoldstrasse 4 80802 München (DE)**

(56) References cited:
| | |
|---|---|
| EP-A- 1 643 248 | WO-A-2006/071696 |
| WO-A2-02/26376 | US-A1- 2002 127 589 |
| US-A1- 2003 059 686 | US-A1- 2003 148 401 |
| US-A1- 2004 109 935 | US-A1- 2004 203 056 |
| US-A1- 2005 008 674 | US-A1- 2005 059 169 |
| US-A1- 2005 214 855 | |

SEQUENCE LIST

<110> Samsung Electronics Co. Ltd.

<120> Patterned spot microarray using photocatalyst and method of manufacturing the same

<130> EP46801Fz106

<140> not yet assigned
<141> herewith

<150> KR 10-2006-0069797
<151> 2006-07-25

<160> 2

<170> KopatentIn 1.71

<210> 1
<211> 19
<212> DNA
<213> Artificial Sequence

<220>
<223> probe polynucleotide coupled with NH2-(CH2)- group at it's 5' terminal

<400> 1
gatggaaggt gctgggagc        19

<210> 2

<211> 19
<212> DNA
<213> Artificial Sequence

<220>
<223> target polynucleotide labelled with cy-3 at it's 3' terminal

<400> 2
gctcccagca ccttccatc        19

**Description**

BACKGROUND OF THE INVENTION

1. Field of the Invention

The present invention relates to a method of preparing an integrated spot microarray by patterning a formed spot using a photocatalyst and a microarray manufactured using the method.

2. Description of the Related Art

In order to detect a biomolecule (DNA, RNA, or the like) existing in a sample from a living body, a DNA microarray (also called a DNA chip) is used. Using the DNA microarray, several hundreds up to tens of thousands of biomolecules can be detected simultaneously and nucleotide sequences thereof can be determined at the same time. In a DNA microarray, several hundreds through several tens of thousands of DNA detection points (spot part) are arranged evenly distributed on a glass substrate or a silicon substrate having a size of several square centimeters to several tens of square centimeters. At each DNA detection point, one type of single-chained nucleic acid polymer (gene fragment) having a specific nucleotide sequence is immobilized as a probe (detector). That is, a plurality of different nucleic acid probes is arranged on the DNA microarray. Then, if an aqueous solution of sample nucleic acids, which are labeled with a fluorescent material, is poured onto the DNA microarray, sample nucleic acids hybridize with probes of the DNA microarray, if the sequence of the sample nucleic acid is complementary to the sequence of a probe. After removal of the sample solution, only sample nucleic acids, which have hybridized with a probe of the DNA microarray, are captured on the microarray. By detecting fluorescence of the fluorescent material labeling the sample nucleic acids bound to a probe on the DNA microarray, it is determined whether a target nucleic acids exists among the sample nucleic acids.

DNA microarrays can be classified according to the production method into two types of microarrays, that is, a photolithography type and a spotting type.

The photolithography type is manufactured by synthesizing the different DNA probes (oligonucleotide) one nucleotide at a time onto a substrate (or sheet) by a photolithographic method used in a manufacturing process of a semiconductor integrated circuit. Said production technique can be used to form a DNA microarray having a high density of DNA detection points (U.S. Patent Nos. 5,744,305 and 5,445,934).

Meanwhile, the spotting type uses a substrate (or sheet), such as a glass slide, which is coated with a solidified product (for example, aminosilane). In the spotting type, various droplets each including a prepared probe DNA are spotted onto the substrate one by one, the droplets are dried, and then a method of forming a DNA detection spot is carried out to immobilize the probes on the substrate (U.S. Patent No. 5,87522).

These two kinds of DNA microarrays have different properties. The DNA microarray of the photolithography type ensures a high measuring sensitivity and reproducibility, and it can be used for analyzing a single nucleotide polymorphism (SNP). However, an individual mask is required in order to elongate the growing chain of the probes by a specific single nucleotide. Since there are four kinds of nucleotides, that is, A, T, G and C, the DNA microarray of the photolithography type requires at least four masks for elongating all probes of a microarray by one nucleotide. For example, up to 80 masks may be required when probes having 20 bases are synthesized. Since each mask is expensive, the manufacture of a DNA microarray using the photolithography method is expensive. Thus, only a few research institutions use the DNA microarray of the photolithography type recently.

The DNA microarray of the spotting type uses a method of spotting a droplet containing a pre-synthesized DNA probe onto a substrate, which droplet is then dried. Thus, the manufacture of a DNA microarray using this method is less expensive than the photolithography type. On the other hand, the DNA microarray of the spotting type is suboptimal in terms of the density and uniformity of the DNA probes immobilized on the substrate. Recent marketed products, which are manufactured using a DNA microarray of the photolithography type, have a spot size of about 5 $\mu$m. Products which are manufactured using a DNA microarray of the spotting type, have a minimum spot size of about 60 $\mu$m (SMP2 pin). Accordingly, in order to spot biomolecule probe with a high density, it is required to reduce the spot size.

A process of immobilizing biomolecules onto a microarray substrate is known in the art. Examples of immobilization methods include a method using photolithography and a method using spotting. According to the method using photolithography, a polynucleotide microarray may be manufactured by repeating an operation of exposing a source of energy to a certain region on the surface of a substrate, which region is coated with monomers protected by removable groups, so as to remove the removable protective groups and coupling the monomers. In this case, a polynucleotide immobilized on the polynucleotide microarray is synthesized by extending monomers one at a time. Meanwhile, according to the spotting method, a microarray is manufactured by immobilizing pre-synthesized polynucleotides on the substrate at fixed positions determined by the region, where droplets of a solution containing respective polynucleotides have been deposited. These methods of preparing the polynucleotide are disclosed in, for example, U.S. Patent Nos. 5,744,305, 5,143,854, and 5,424,186. These references concern a polynucleotide microarray and a method of preparing the same.

EP 1 643 248 A refers to a substrate for a biomolecule microarray having one or more spots for immobilizing a microarray. Said spots protrude from the surface of the substrate and have a flat surface for spotting the biomolecule on top thereof and lateral surface around the protruding spot having a roughly V-shaped bottom surface. At least the surface of the substrate around the protruding spot part and the flat surface for spotting are comprised of or coated with an electrically conductive substance, such as $SnO_2$.

US 2003/148401 A1 refers to a microarray substrate having a plurality of microfeatures that provide a high surface area and are open to provide ready access to fluids and components therein. In one embodiment, the microfeatures are formed of, e.g., an inorganic oxide such as zinc oxide.

WO-A-02126376 relates to a method for generating high density arrays including a printing and an illumination step. In the printing step, spots of reagent solution containing receptor molecules, such as nucleic acids, are applied to a solid support in a desired pattern. In the illumination step, the spots are irradiated either through a mask or alternatively using mirrored laser technology to immobilize the receptor molecule to the solid substrate. The immobilized reagent spot has a smaller diameter than the original printed spot. After the illumination step, the reagent that has not been immobilized may be removed by a wash step and the process of printing, illumination and washing may be repeated, although offset from the original pattern, to manufacture a high-density area.

In the present invention a photocatalyst layer is formed by coating a photocatalyst such as TiO2 onto a substrate. Irradiating the photocatalyst with light results in the formation of radicals, which are able to oxidize organic compounds and initiate chemical reactions. A microarray of the spotting type having a reduced spot size can be manufactured by photolithographically patterning existing spots to complete manufacture of the present invention.

SUMMARY OF THE INVENTION

The present invention provides a method of preparing a patterned spot microarray using a photocatalyst by which method the size of the patterned spot can be reduced.

A method of preparing a patterned spot microarray using a photocatalyst comprises the steps of coating the photocatalyst on a substrate to form a photocatalyst layer; coating a material comprising a functional group to be connected to a biomolecule on the photocatalyst layer to form an organic thin film layer; spotting the biomolecule on the organic thin film layer; and arranging a photomask comprising plurality of openings above a spot of the biomolecule to divide one spot into patterned spots by irradiating light through the respective openings in the photomask.

In a preferred embodiment, the photocatalyst is TiO2, ZnO, SnO2, SrTiO3, WO3, B2O3, or Fe2O3.

The photocatalyst layer may be formed using chemical vapor deposition (CVD), physical vapor deposition (PVD), sputtering or a sol-gel method.

The functional group to be connected to the biomolecule can be an amine group, a carboxyl group, an epoxy group, or a sulfur group.

Preferably, the material comprising a functional group to be connected to the biomolecule is γ-aminopropyltriethoxy silane (GAPS).

In another embodiment, the photocatalyst layer may have a thickness such that constructive interference occurs between first reflective light of irradiated excitation light reflected from the substrate and second reflected light of irradiated excitation light reflected from the photocatalyst layer. The photocatalyst layer can have a thickness in the range of 350 to 1100 Å.

Preferably, the diameter of the patterned spot is 100 μm or less, and the shape of the patterned spot is a circle, a lozenge, a regular tetragon, a rectangle or a star.

The substrate can be any one selected from the group consisting of glass, a silicon wafer, fused silica, gold, silver, copper, platinum, polystyrene, poly(methacrylate) and polycarbonate.

The present invention also provides a microarray manufactured using the method according to the present invention.

BRIEF DESCRIPTION OF THE DRAWINGS

The above and other features and advantages of the present invention will become more apparent by describing in detail exemplary embodiments thereof with reference to the attached drawings in which:

FIG. 1 is a diagram illustrating a microarray according to an embodiment of the present invention;
FIG. 2 is a diagram illustrating the photomask arranged on top of a spot of the microarray of FIG. 1;
FIG. 3 is a diagram illustrating the effect of varying the thickness of an oxide layer pattern on fluorescence intensity; and
FIG. 4 is a diagram illustrating fluorescence intensity according to a hybridization of a target nucleic acid.

DETAILED DESCRIPTION OF THE INVENTION

The present invention will now be described in more detail with reference to the accompanying drawings, in which exemplary embodiments of the invention are shown. These embodiments are provided so that this disclosure will be thorough and complete, and will fully convey the concept of the invention to those skilled in the art.

According to an aspect of the present invention, there is provided a method of preparing a patterned spot microarray using a photocatalyst, the method comprising: coating the photocatalyst onto a substrate to form a photocatalyst layer; coating a material onto the photocatalyst layer to form a thin film layer, said material comprising a functional group to be linked to a biomolecule when the photocatalyst is activated by light; spotting a biomolecule onto the organic thin film layer; and irradiating light through a photomask having a defined pattern onto a biomolecule spot to pattern the spot by photocatalytically immobilizing the biomolecule according to claim 1. According to the present invention, photocatalytically immobilizing refers to an immobilization of the biomolecules to the microarray, which immobilization is induced by light.

In the method according to an embodiment of the present invention, areas of the photocatalyst are irradiated with light, such as ultra violet light, which areas are defined by the apertures of the photomask. Upon irradiation, the photocatalyst generates radicals, which are capable of oxidizing the functional group the material of the thin film layer, which may preferably be an organic compound. The photocatalyst may be Ti02, ZnO, Sn02, SrTiO3, W03, B2O3, or Fe2O3, preferably, TiO2. When light is irradiated onto a microarray through the photomask according to the present invention, radicals are thus created only in the irradiated regions of the photocatalyst layer. The radicals produced hence oxidize the functional groups of the compound in the, preferably organic, thin film layer formed onto the photocatalyst layer substantially in the irradiated regions. The oxidized functional group in the organic compound, in turn, reacts with the biomolecules in a manner immobilizing the biomolecules. Thereby, a droplet of a biomolecule solution, which is initially spotted onto the organic thin film layer, is photolithographically patterned into a patterned spot thereby reducing the size of the spot to a level comparable to the level of photolithographic arrays. In the present invention, "spot" refers to a certain region where an individual polynucleotide is immobilized.

In the method according to the current embodiment of the present invention, the photocatalyst layer can be formed by chemical vapor deposition (CVD), physical vapor deposition (PVD), sputtering, a sol-gel method, or the like, but the method is not limited thereto. A metal oxide, like the above-mentioned preferred photocatalysts, can be deposited onto a substrate using a known technique.

CVD is a method of thin film deposition, in which gaseous elements are chemically reacted onto a substrate, such as a wafer and the like, to form a stable solid thin film. Low pressure CVD is a method of thin film deposition in a vacuum. The deposition speed can be increased by forming a vacuum inside a chamber, in which the thin film is deposited, and the density of a by-product, which can be generated by the presence of air, can be lowered. Various thin films such as a silicon nitride (SiN) thin film and a silicon oxide ($SiO_2$) thin film, a polycrystalline silicon (Poly-Si) thin-film, or the like can be deposited using CVD.

PVD is a method that is used in thin film formation. PVD has recently been favored as a means of surface curing, since a thin film can be more simply obtained by low temperature treatment that cannot be performed using other methods. Examples of PVD methods include an evaporation deposition method that does not use ions, a sputtering method that uses ions, an ion plating method, an ion implantation method, an ion beam mixing method and the like. Metal is evaporated when heated in a vacuum, and this principle is applied in the evaporation deposition method.

When a particle having high energy collides with a target material, atoms or molecules are ejected from the target material. This phenomenon is called sputtering. In sputtering, a target material and a substrate form an anode and a cathode, respectively, and then a high pressure of about $10^{-2}$ Torr is applied between the anode and cathode under Ar atmosphere. As a result, Ar gas around the anode is ionized to $Ar^+$ and then the $Ar^+$ is directed to and collides with the anode. Molecules or atoms of the target material are ejected from the anode by the $Ar^+$ ion bombardment and are bound to the substrate, that is, form a thin film on the cathode. Examples of sputtering include DC sputtering, RF sputtering, bias sputtering, magnetron sputtering and the like, and in particular, magnetron sputtering is a high speed sputtering method and favored in various fields.

In a sol-gel method, metal oxide having a colloid form is prepared through hydrolysis reaction of a metal halide or alkoxide. The sol-gel method is an exemplary method of preparing a coating solution of titanium dioxide ($TiO_2$). Physical characteristics of the titanium dioxide prepared, such as a particle size, a crystalline property and the like are affected by the kind of alkoxide used, reaction conditions (temperature, pH, a mol ratio between reactants, etc.) and the like.

In the method according to the present invention, the organic thin film layer may be much thinner than the photocatalyst layer. The organic thin film layer is oxidized by the active oxygen or a hydroxyl radical generated when and where the photocatalyst is irradiated with light, and includes a material, preferably an organic material, having a functional group. The functional group can be linked to a corresponding biomolecule when the photocatalyst is activated by light, e.g. generated active oxygen or a hydroxyl radical. The functional group may be an amine group, a carboxyl group, an epoxy group, a sulfur group, or the like, but is not limited thereto. The material having the functional group to be linked to the biomolecule may be γ-aminopropyltriethoxy silane (GAPS), or the like, but is not limited thereto. Immobilizing polynucleotides to the GAPS occurs in that the amine functional group present in GAPS binds to DNA. Generally, for immobilizing polynucleotides to the GAPS, linkers such as amine-C6 linker, PEG (poly ethylene glycol), and ethylene diamine are used or the DNA end is modified with COOH or an epoxy functional group.

In the method according to the present invention, the photocatalyst layer may have a thickness at which constructive interference occurs between first reflected light, which is irradiated excitation light reflected from the surface of the substrate and second reflected light, which is irradiated excitation light reflected from the surface of the photocatalyst layer. In the present invention, the "thickness at which constructive interference occurs" is not a fixed value. In fact, the "thickness at which constructive interference occurs" comprises any thickness in a predetermined range, at which constructive interference occurs between the first reflected light and the second reflected light, wherein constructive interference results in an increase of the light intensity of reflected light compared to the intensity of incident light.

In the method according to the present invention, the index of refraction of the substrate may be greater than the index of refraction of the photocatalyst layer. Constructive interference occurs under the condition that a path difference, $2n\sin\theta d$, between the first reflected light and the second reflected light is as follows:

$$2n\sin\theta d = (2m+1)\lambda/2$$

In the equation, d is the thickness of the photocatalyst layer, n is the index of refraction of the photocatalyst, θ is an angle of incidence of the incident light, m is an integer (m=0,1,2,3...), and λ, is a wavelength. According to the equation, when a path difference of the first reflected light and the second reflected light is an odd multiple of half a wavelength, constructive interference occurs. If the wavelength λ is 532 nm, the reflectance n is 2.3 (of $TiO_2$), m is 0, and the angle of incidence θ is 90°, constructive interference occurs when the thickness of the $TiO_2$-photocatalyst layer d is 570 Å.

Destructive interference occurs under the condition $2n\sin\theta d = (2m)\lambda/2$. Here, if the wavelength λ is 532 nm, the index of refraction n is 2.3, the incidence angle θ is 90°, and m is 1, destructive interference occurs when the thickness of the $TiO_2$-photocatalyst layer d is 1156 Å. Accordingly, the constructive interference occurs when the thickness of the photocatalyst layer d is in the range of 350 to 1100 Å. Thus, a signal can be detected without destructive interference occurring. The thickness of the photocatalyst layer may be in the range of 350 through 1100 Å. When the thickness of the photocatalyst layer is out of the range, constructive interference of the light does not occur, thus, signal-detecting sensitivity is deteriorated.

In the method according to the present invention, the diameter of the patterned spot may be 100 μm and less. In contrast, since the size of the spot integrated using current sputtering is about 100 μm, the spot cannot be patterned to have a smaller size by sputtering.

In the method according to the present invention, the shape of the patterned spot may be a circle, a lozenge, a regular tetragon, a rectangle, a star, or the like, but is not limited thereto. The shape of the patterned spot may be determined according to the shape of a photomask, namely the shape of the apertures in the photomask.

In the method according to present invention, the substrate may be any material, which may be formed as a layer, including polymers, metals, ceramics, oxides, and the like. An array is formed on or supported by the surface of the substrate. The substrate may be an inorganic material, an organic material, a composite, or a polymer. Examples of the substrate include, but are not limited to, glass, a silicon wafer, fused silica, gold, silver, copper, platinum, polystyrene, poly(methylacrylate), and polycarbonate. The surface of the substrate may be modified, and adsorption, chemical reactions, or physical interactions may occur between the modified surface and an element of an array, which element can support, promote, or catalyse the formation of the array.

In the method according to the embodiment of the present invention, the biomolecule may include a nucleic acid such as DNA, RNA and peptide nucleic acid (PNA), a protein such as peptide and polypeptide, and/or a polysaccharide, but is not limited thereto.

The present invention also provides a microarray manufactured using the method according to the present invention.

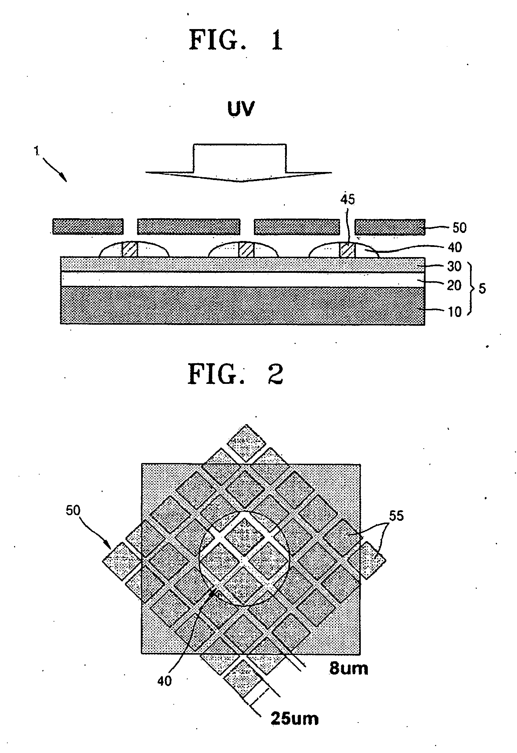

FIG. 1 is a diagram illustrating a microarray 1 according to an embodiment of the present invention. Referring to FIG. 1, the microarray 1 includes a microarray substrate 5 and a patterned spot 45, where biomolecule are immobilized to the microarray substrate 5. The microarray substrate 5 comprises a substrate 10, a photocatalyst layer 20, and an organic thin film layer 30. A photomask 50 is arranged above a biomolecule spot 40 deposited on the microarray substrate 5, and then a patterned spot 45 is formed by irradiating UV light through the photomask 50 onto the array structure. The size and shape of the patterned spots 45 are determined by the size and the shape of the openings 55 in the photomask 50. FIG. 2 illustrates the photomask 50 comprising a plurality of regularly distributed, rectangular openings 55 arranged above one biomolecule spot 40 of the microarray 1 of FIG. 1. Referring to FIG. 2, one spot 40 can be divided into four patterned spots 45 by irradiating light through the respective openings 55 in the photomask 50. Since one spot is divided into four spots which can obtain four pieces of data, a final integration rate is increased by 4 times per unit area. In addition, if a spot 50, whose data cannot be extracted owing to a defect inside the spot 40, is divided partially into patterned spots 45 according to the present invention, not all of the patterned spots 45 have the defect inside the initial spot 40. Thus, patterning the spot makes is possible to extract data from at least the patterned spots 45, which do not have the defect. The conventional method of patterning is complicated and it needs various operations. However, according to the current embodiment of the present invention, the organic layer may be easily patterned only using two operations (UV exposure and cleaning). Accordingly, the method according to the current embodiment of the present invention can be used in all fields, in which a spotted biomolecule can be easily patterned by oxidation induced by UV light using a photomask, preferably, in patterning a microarray or an insulating layer.

The present invention also provides a microarray substrate comprising a photocatalyst layer coated onto the substrate, and an thin film layer, preferably composed of an organic material, coated onto the photocatalyst layer, said thin film layer comprising a material having a functional group to be linked to a biomolecule when the photocatalyst is activated by light. On the microarray substrate, the microarray may be formed by spotting a plurality of biomolecules onto the organic thin film layer and irradiating light through a photomask having a defined pattern onto a biomolecule spot to pattern the spot by photocatalytically immobilizing the biomolecule. The biomolecule may include nucleic acids such as DNA, RNA and peptide nucleic acid (PNA), protein such as peptide and polypeptide, and polysaccharide, but is not limited thereto.

The microarray of the present invention thus comprises: the above microarray substrate, wherein a biomolecule is linked to the functional group of the material forming the thin film layer.

The present invention will now be illustrated in further detail with reference to the following examples.

Example 1: Preparation of a microarray substrate

A substrate composed of silicon was used. An oxide layer (either $SiO_2$ or $TiO_2$) was formed onto the substrate by thermal oxidation using a furnace SVF-200 apparatus (available from Seltron Inc.) having a defined thickness. The thickness of the titanium dioxide layer was about 370 Å (sample A), about 530 Å (sample B) and about 600 Å (sample C). The thickness of the silicon dioxide layer was about 1000 Å (sample D).

The thickness of the oxide layers formed was measured using NANOSPEC Model AFT 200 (available from NANOMETTICS Inc.). The NANOSPEC apparatus uses the principle that, when light was irradiated onto a silicon wafer, some of the light is reflected by the oxide layer while some of the light passed through the oxide layer and is reflected by the silicon substrate. The phase difference between the light reflected by the oxide layer and the light reflected by the silicon substrate can be used to determine the thickness of the oxide layer. The thickness of the oxide layer was measured by placing the silicon wafer on a sample stage of the NANOSPEC apparatus. The thickness was measured at 5-6 points on the wafer and an average value was derived therefrom to be used for the mean coating thickness. All experiments were performed in a clean room-class 1000 from which most dust particles were removed.

A solution of 20% (vol/vol) GAPS (γ-aminopropyltriethoxy silane) in ethanol was spin coated on the substrate having the silicon dioxide or titanium dioxide layer using a spin coater model CEE 70 (available from CEE Inc.). The spin coating was performed by performing an initial coating at 500 rpm for 10 sec and a main coating at 2,000 rpm fog 10 sec. After the spin coating was completed, the substrate was fixed to a Teflon® wafer carrier and incubated for 13 minutes at a temperature of 20 °C and a relative humidity equal to 45%, and then cured at 120 °C for 40 minutes. The cured substrate was immersed in water for 10 minutes, ultrasonically washed, immersed again in water for 10 minutes, and then spin-dried. The dried substrate was cut to have a square or rectangular shape and used in the experiments. All experiments were performed in a clean room-class 1000 from which most dust particles were removed.

10 mM of Fluorescein was dip-coated onto each of the oxide layer coated substrates. First, Fluorescein was dissolved in dimethyl formamide (DMF) solution to prepare an immersion solution (10 mM of Fluorescein). The immersion solution and the substrate were placed in a reaction container and reacted at 40 °C for 120 minutes. After completing the reaction, the substrate was removed from the immersion solution, and cleaned three times using DMF for 10 minutes each, and then cleaned three times using methanol for 10 minutes each. Thereafter, the substrate was dried, and then the amount of Fluorescein reacted with each of the substrates was determined using GenePix 4000B scanner (available from Axon Inc.).

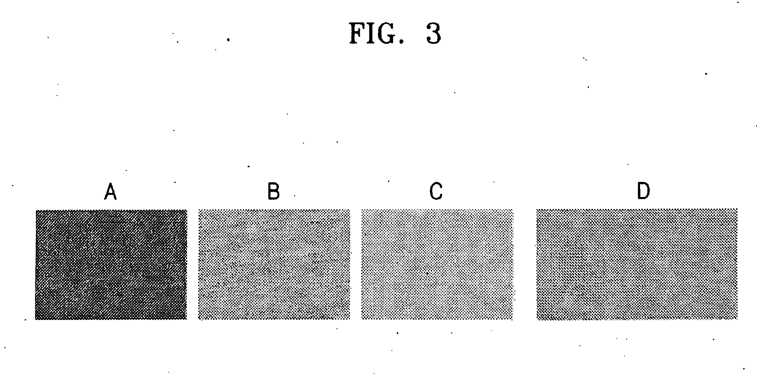

The obtained results are illustrated in FIG. 3. FIG. 3 is a diagram illustrating an effect of various thicknesses of an oxide layer pattern on fluorescence intensity. Since, theoretically, light is maximally constructively interfered using a $SiO_2$ (reflectance = 1.46) layer having a thickness of 1000 Å, light should be maximally constructively interfered using a $TiO_2$ layer (reflectance = 2.36) having a thickness of 570 Å. The type of oxide layer used, the thickness of the oxide layer, the fluorescence intensity and the relative ratio of fluorescence intensity of each panel A to D formed in relation to the fluorescence intensity of panel D are shown in Table 1.

Table 1

| Panel | Oxide layer | Thickness of layer (Å) | Fluorescence intensity | Relative ratio |
|---|---|---|---|---|
| A | $TiO_2$ | 370 | 475 | 0.04 |
| B | $TiO_2$ | 530 | 2800 | 0.25 |
| C | $TiO_2$ | 600 | 4300 | 0.39 |
| D | $SiO_2$ | 1000 | 11000 | 1.00 |

Referring to FIG. 3 and Table 1, when light is constructively interfered on a $TiO_2$ layer having a thickness of 600 Å, the fluorescence intensity is measured to be 40 % of the extremely high fluorescence intensity of a $SiO_2$ layer having a thickness of 1000 Å as a control group. Accordingly, $TiO_2$ can be used for the measurement of the fluorescence intensity in a microarray instead of $SiO_2$.

Example 2: Preparation and hybridization of a polynucleotide microarray

In this Example, probe polynucleotides were immobilized on a microarray substrate comprising the $SiO_2$ oxide layer having a thickness of 1,000 Å and substrate comprising the $TiO_2$ oxide layer having a thickness of 600 Å, panels D and C prepared in Example 1, respectively. The microarray was manufactured in that spots containing the probe polynucleotides were deposited on the microarray substrates, which spots were then patterned using a photomask and UV-radiation. Thereafter, labeled target nucleic acids were hybridized to the probes, and then the fluorescence intensity was measured to investigate the effect of the patterned spot on the fluorescence intensity as described in detail below

First, as described in Example 1, the oxide layer was obtained and probe polynucleotides were immobilized thereon. The immobilization of the probe polynucleotides as patterned spots was achieved by depositing the probe polynucleotides onto the substrate using a spotting process and fixing the probe polynucleotides to the substrate photolithographically. The probe polynucleotides were added to a 25 mM $NaHCO_3$ (pH = 9.0) solution, and the resulting solution was left at 37 °C for 1 hour. This spotting solution was spotted onto the substrate, and then the spotted substrate was left in a wet chamber at 70 °C under a relative humidity of 40% for 1 hour. Then, a process required for background control was performed. That is, in order to prevent that target nucleic acids bind to the glass surface of the substrate, amine groups in unspotted positions of the substrate surface were allowed to be negatively charged by treatment with succinyl anhydride. Then, the substrate was stored in a drying machine. To pattern the spot, next, UV light was irradiated for 10 minutes onto the spot through a patterned photomask comprising 25 μm openings. The target polynucleotide, which is complementary to the probe polynucleotide immobilized in the patterned spot of the array and which is labeled with Cy-3, was hybridized with the probe polynucleotide of the prepared microarray, i.e. the DNA chip, and then the fluorescence was measured at a wavelength of 532 nm.

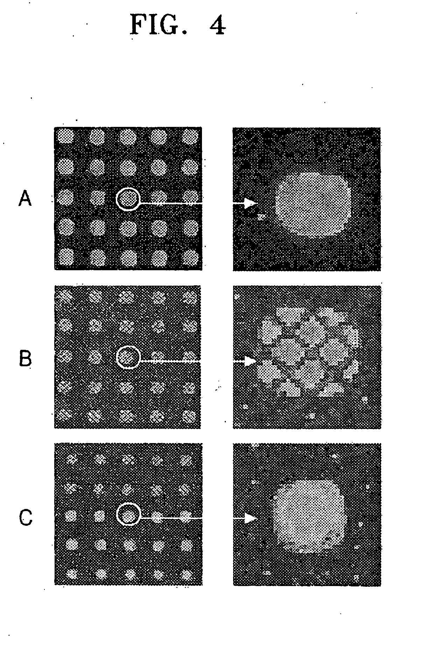

The obtained results are illustrated in FIG. 4. FIG. 4 is a diagram illustrating the fluorescence intensity according to the hybridization of the target nucleic acids to the probes of the array. Referring to FIG. 4, the three right panels show the encircled spot of the corresponding left panel enlarged. Panel A refers to an array comprising $SiO_2$ as the oxide layer, which was not subjected to the photolithographic patterning according to the present invention. Panel B of Fig. 4 refers to an array comprising $TiO_2$ as the oxide layer, in which all spots were photolithographically patterned according to the present invention. Panel C of Fig. 4 refers to an array comprising $TiO_2$ as the oxide layer, in which the spots of two upper lines were photolithographically patterned according to the present invention while the lower three lines of spots were not subjected to UV-light.

Referring to FIG. 4, oxide layer patterns have a thickness of 1,000 Å ($SiO_2$) and 600 Å ($TiO_2$), in which con-structive interferences occurs, were manufactured. The resulting products were coated with GAPS and then spotted with probe oligonucleotide (SEQ ID NO: 1: 5'-GATGGAAGGTGCTGGGAGC-3'). Thereafter, the spotted probe droplets were patterned by irradiating UV-light through a photomask onto the droplets. A target oligonucleotide (SEQ ID NO: 2: 5'-GCTCCCAGCACCTTCCATC-3') labeled with Cy-3 was brought into contact with the probe nucleotides immobilized on the array in order to allow hybridization. The fluorescence intensity of the resulting products was measured at 532 nm. The mean fluorescence intensity of a spot, the mean spot size and the relative ratio, i.e. the mean fluorescence intensity in relation to the mean fluorescence intensity of the control panel A, are shown in Table 2 below.

## EP 1 882 520 B1

Table 2

| Panel | Oxide layer | Thickness of layer (Å) | Mean fluorescence intensity | Mean spot size ($\mu$m) | Relative ratio |
|---|---|---|---|---|---|
| A | $SiO_2$ | 1000 | 23068 | 115 | 1.00 |
| B | $TiO_2$ | 600 | 8229 | 25 | 0.35 |
| C | $TiO_2$ | 600 | 6785 | 95 | 0.29 |

Referring to FIG. 4 and Table 2, the fluorescence intensity of $TiO_2$ according to the present invention is slightly smaller than that of $SiO_2$. However, the mean size of the patterned spots is 25 $\mu$m in B panel. It can be seen that the mean spot size of B panel is remarkably reduced in comparison with 115 $\mu$m of the A panel and 95 $\mu$m of the C panel, in which patterned and unpatterned spots coexist.

Accordingly, it can be seen that the spot size of a microarray is remarkably reduced using $TiO_2$ constituting the photocatalyst according to the present invention.

According to the present invention, a spot having a size of less than 60 $\mu$m, which is the minimum size formable using conventional spotting methods, can be manufactured. Thus, more integrated chip having a high spot density can be manufactured. The shape of the patterned spot, which is determined by the spotting in the conventionally spotting method, can be easily regulated and adjusted using an appropriate photomask according to the present invention. Patterning can be easily performed in the present invention using only one step of UV exposure, unlike the conventional photolithography method, without the need of using a photoresist or performing further steps of baking, coating, or the like. Furthermore, damaged spots that cannot be used as a data in a conventional pin array spot, can be partially used in after patterning such damaged spots according to the present invention. According to the present invention, the data loss due to spot damage can thus be minimized.

## Claims

1. A method of preparing a patterned spot microarray using a photocatalyst, the method comprising:

    coating the photocatalyst on a substrate to form a photocatalyst layer;
    coating a material comprising a functional group to be connected to a biomolecule on the photocatalyst layer to form an organic thin film layer;
    spotting the biomolecule on the organic thin film layer; and
    arranging a photomask comprising a plurality of openings above a spot of the biomolecule to divide one spot into patterned spots by irradiating light through the respective openings in the photomask.

2. The method of claim 1, wherein the photocatalyst is $TiO_2$, $ZnO$, $SinO_2$, $SrTiO_3$, $WO_3$, $B_2O_3$, or $Fe_2O_3$.

3. The method of claim 1 or 2, wherein the photocatalyst layer is formed using chemical vapor deposition (CVD), physical vapor deposition (PVD), sputtering or a sol-gel method.

4. The method of any of claims 1 to 3, wherein the functional group to be connected to the biomolecule is an amine group, a carboxyl group, an epoxy group, or a sulfur group.

5. The method of any of claims 1 to 4, wherein the material comprising a functional group to be connected to the biomolecule is γ-aminopropyltriethoxy silane (GAPS).

6. The method of any of claims 1 to 5, wherein the photocatalyst layer has a thickness such that constructive interference occurs between first reflective light of irradiated excitation light reflected from the substrate and second reflected light of irradiated excitation light reflected from the photocatalyst layer.

7. The method of claim 6, wherein the photocatalyst layer has a thickness in the range of 350 to 1100 Å.

8. The method of claim 1, wherein the diameter of the patterned spot is 100 μm or less.

9. The method of any of claims 1 to 8, wherein the shape of the patterned spot is a circle, a lozenge, a regular tetragon, a rectangle or a star.

10. The method of any of claims 1 to 9, wherein the substrate is any one selected from the group consisting of glass, a silicon wafer, fused silica, gold, silver, cupper, platinum, polystyrene, poly(methacrylate) and polycarbonate.

11. A microarray manufactured using the method of any of claims 1 to 10.

## Patentansprüche

1. Verfahren zum Herstellen eines gemusterten gedruckten Mikroarray ("patterned spot microarray") unter Verwendung eines Fotokatalysators, wobei das Verfahren umfasst:

    Auftragen des Fotokatalysators auf einen Träger, um eine Fotokatalysatorschicht auszubilden;
    Auftragen eines Materials, das eine funktionelle Gruppe umfasst, die mit einem Biomolekül verbunden werden soll, auf die Fotokatalysatorschicht, um eine organische Dünnfilmschicht auszubilden;

Platzieren der Biomoleküle auf der organischen Dünnfilmschicht; und

Anordnen einer Fotomaske, die eine Vielzahl von Öffnungen aufweist, über einem Tropfen von den Biomolekülen, um den einen Tropfen durch Strahlen von Licht durch die entsprechenden Öffnungen in der Fotomaske in gemusterte Tupfen zu unterteilen.

**2.** Verfahren gemäß Anspruch 1, wobei der Fotokatalysator $TiO_2$, $ZnO$, $SnO_2$, $SrTiO_3$, $WO_3$, $B_2O_3$ oder $Fe_2O_3$ ist.

**3.** Verfahren gemäß Anspruch 1 oder 2, wobei die Fotokatalysatorschicht durch chemische Gasabscheidung (CVD), physikalische Gasabscheidung (PVD), Sputtern oder in einem Sol-Gel-Verfahren ausgebildet wird.

**4.** Verfahren gemäß einem der Ansprüche 1 bis 3, wobei die funktionelle Gruppe, die mit dem Biomolekül verbunden werden soll, eine Amingruppe, eine Carboxylgruppe, eine Epoxygruppe oder eine schwefelhaltige Gruppe ist.

**5.** Verfahren gemäß einem der Ansprüche 1 bis 4, wobei das Material, das eine funktionelle Gruppe, die mit dem Biomolekül verbunden werden soll, umfasst, $\beta$-Aminopropyltriethoxysilan (GAPS) ist.

**6.** Verfahren gemäß einem der Ansprüche 1 bis 5, wobei die Fotokatalysatorschicht eine Dicke hat, die konstruktive Interferenz zwischen erstem reflektiertem Licht des ausgesandten Lichtstrahls, das am Träger reflektiert wird, und zweitem reflektierten Licht des ausgesendeten Lichtstrahls, das an der Fotokatalysatorschicht reflektiert wird, ermöglicht.

**7.** Verfahren gemäß Anspruch 6, wobei die Fotokatalysatorschicht eine Dicke im Bereich von 350 bis 1100 Å aufweist.

**8.** Verfahren gemäß Anspruch 1, wobei der Durchmesser von den gemusterten Tupfen 100 $\mu$m oder kleiner ist.

**9.** Verfahren gemäß einem der Ansprüche 1 bis 8, wobei die Form der gemusterten Tupfen ein Kreis, ein Robus, ein gleichmäßiges Viereck, ein Rechteck oder ein Stern ist.

**10.** Verfahren gemäß einem der Ansprüche 1 bis 9, wobei der Träger ausgewählt ist aus der Gruppe bestehend aus einem Träger aus Glas, einem Silikonwafer, Quarzglas, Gold, Silber, Kupfer, Platin, Polystyrol, Poly(methacrylat) und Polycarbonat.

**11.** Microarray hergestellt unter Verwendung des Verfahrens gemäß einem der Ansprüche 1 bis 10.

## Revendications

**1.** Procédé de préparation d'un microréseau de taches structurées à l'aide d'un photocatalyseur, ledit procédé consistant à :

appliquer le photocatalyseur sur un substrat pour former une couche de photocatalyseur ;

appliquer un matériau, comprenant un groupe fonctionnel à relier à une biomolécule, sur la couche de photocatalyseur pour former une couche d'un mince film organique ;

déposer en taches la biomolécule sur la couche du mince film organique ; et

installer un photomasque comprenant une pluralité d'ouvertures au-dessus d'une tache de la biomolécule pour diviser une tache en taches structurées par irradiation de lumière à travers les ouvertures respectives du photomasque.

**2.** Procédé selon la revendication 1, dans lequel le photocatalyseur est $TiO_2$, $ZnO$, $SnO_2$, $SrTiO_3$, $WO_3$, $B_2O_3$, ou $Fe_2O_3$.

**3.** Procédé selon la revendication 1 ou 2, dans lequel la couche de photocatalyseur est formée par une méthode de dépôt chimique en phase vapeur (CVD), dépôt physique en phase vapeur (PVD), pulvérisation cathodique ou sol-gel.

**4.** Procédé selon l'une quelconque des revendications 1 à 3, dans lequel le groupe fonctionnel à relier à la biomolécule est un groupe amine, un groupe carboxyle, un groupe époxy, ou un groupe soufre.

**5.** Procédé selon l'une quelconque des revendications 1 à 4, dans lequel le matériau comprenant un groupe fonctionnel à relier à la biomolécule est le $\gamma$-aminopropyltriéthoxysilane (GAPS).

**6.** Procédé selon l'une quelconque des revendications 1 à 5, dans lequel la couche de photocatalyseur a une épaisseur telle qu'une interférence constructrice se produit entre la première réflexion lumineuse de la lumière d'excitation irradiée réfléchie par le substrat et la seconde réflexion lumineuse de la lumière d'excitation irradiée réfléchie par la couche de photocatalyseur.

**7.** Procédé selon la revendication 6, dans lequel la couche de photocatalyseur a une épaisseur comprise entre 350 et 1100 Å.

**8.** Procédé selon la revendication 1, dans lequel le diamètre de la tache structurée est inférieur ou égal à 100 $\mu$m.

**9.** Procédé selon l'une quelconque des revendications 1 à 8, dans lequel la forme de la tache structurée est un cercle, un losange, un tétragone régulier, un rectangle ou une étoile.

**10.** Procédé selon l'une quelconque des revendications 1 à 9, dans lequel le substrat est un substrat quelconque choisi dans le groupe constitué du verre, d'une tranche de silicium, de la silice fondue, de l'or, de l'argent, du cuivre, du platine, du polystyrène, du poly(méthacrylate) et du polycarbonate.

**11.** Microréseau fabriqué au moyen du procédé selon l'une quelconque des revendications 1 à 10.

# FIG. 1

UV

# FIG. 2

FIG. 3

A          B          C          D

EP 1 882 520 B1

# FIG. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5744305 A
- US 5445934 A
- US 587522 A
- US 5143854 A
- US 5424186 A
- EP 1643248 A
- US 2003148401 A1
- WO 02126376 A
- EP 46801FZ106 A
- KR 1020060069797